# EUROPEAN PATENT APPLICATION

(11) **EP 1 059 352 A1**
(43) Date of publication of application: **13.12.2000**
(21) Application number: 99111107.1
(22) Date of filing: 08.06.1999
(51) Int. Cl.: C12N 5/08

(54) **Long term cell culture of human carcinoma**

(71) Applicant: Oncodia AG, 8032 Zürich (CH)
(72) Inventor: Bodis, Stephan, 5430 Wettingen (CH); Resch, Hildegard, 8048 Zürich (CH)
(74) Representative: Blum, Rudolf Emil Ernst

(57) **Abstract**

A culture medium suitable for culturing human carcinoma cells and for establishing primary and immortalized cell cultures as well as respective methods are described. The medium and methods are especially suitable for human squamous carcinoma of patients with head/neck cancer. The epithelial cell cultures obtained are very useful tools for diagnostic, development of novel and optimization of existing therapies, drug screening, genetic engeneering, etc.

## Description

### Technical field

The present invention concerns a culture medium suitable for the continuous proliferation of human cells in monolayer cultures, as well as a method for culturing human carcinoma cells, in particular human squamous carcinoma cells of patients with head/neck cancer.

### Background art

The establishing of cell cultures of human cells, be it for diagnosis, the development of novel therapeutic methods, the best treatment for one specific patient, the best combination of treatments for one specific patient, drug screening, or as host cells for protein production, is very much desired. However, the hitherto known methods only allow very few cycles and have a quite low reliability due to changes in the cell morphology and/or a high rate of cell death. It is thus still very much desired to get an improved culturing method.

Cancer, such as human squamous cell carcinoma of the head and neck area, is still a challenge for the clinician. Despite advances in surgery, radiotherapy and chemotherapy there has been little improvement in survival over the last 25 years ¹. Therefore reliable prognostic factors and new therapeutic modalities are needed. In vitro systems, in which carcinoma such as squamous cell carcinoma of the head and neck could be studied under carefully controlled conditions, can be a useful tool. Maintaining primary cultures of human carcinoma cells, such as epithelial cells, is difficult ^{2,3,4} . Cultures are often lost due to bacterial/fungal contamination, massive tumor cell necrosis, tumor cell senescence or fibroblast overgrowth.

In vitro culture systems that allow extended growth of tumor cells, e.g. epithelial tumor cells, are a powerful tool for the study of carcinogenesis and cancer therapy, but also for the development of new therapies and drugs. Conditioned culture systems for studies of basic cell biology have been established that permit the passage of cells and development of cell lines in a variety of cell types. However, to accomplish this, the cells must undergo a selection process and only a small number of the original cell population continues to grow; consequently their application in the investigation of disease-specific mechanisms is limited. In order to avoid cell selection as well as other artefacts that may be induced by long term culture, attempts have been made to improve the conditions for primary cultures of human epithelial cells ^{5,6}. The aim was to get cultures that are more representative of the primary tumor, due to conservation of tumor cell heterogeneity.

Past studies for cell biology of epithelia have utilized primary cultures ^{7,8} , continuous lines from a variety of species and organs ⁹ and feeder layers ¹⁰ . Only few cell culture systems have been reported in which human epithelial cells have undergone repetitive passages. However, attempts to establish tumor cell lines derived from human primary tumor tissue such as head/neck area (tongue, larynx and pharynx) have not been successful so far. The main problems observed in non-selective media containing fetal bovine serum in usual quantities were fibroblast overgrowth, inhibition of cell growth, progressive deterioration of cell morphology, and cell differentiation with loss of cell proliferation.

However, recent progress in the design of culture media allows epithelial cell growth. Fibroblasts do not grow well in such media optimized for epithelial cells. One of the critical requirements for growth of epithelial cells is the prevention of terminal differentiation. Epithelial cells are therefore grown in low (0.09 mM) concentration of calcium ¹¹ using trace amounts (0.01 % v/v) of serum and specific hormones and growth factors ^{12,13}.

A number of serum-free media have been developed that selectively stimulate the growth of epithelial cells ^{14,15} . But even when using low concentrations of fetal bovine serum (2-4 %) in these media, the attachment of epithelial cells remained poor and the use of extracellular matrix (collagen, fibronectin) was necessary ^{16,17}.

Despite these improvements, primary culture systems for malignant epithelial cells have been limited due to small tissue samples, fungal contamination, poor epithelial cell yield from heterogeneous tissue particles and the limited proliferative capacity in vitro.

Thus, a lot of efforts have been made to screen for primary cell culture conditions that will enable to culture large numbers of malignant epithelial cells of head/neck area, maintaining their initial phenotype ^{6,18,19,20}, however, so far with unsatisfying results.

It is therefore still desired to get cell culture conditions that enable the culturing of large varieties of phenotypes of malignant cells, in particular malignant epithelial cells of head/neck area in such a way that they conserve their initial phenotype. Besides of primary cell cultures that are especially important for diagnosis, development of new treatments and optimization of already known treatments or combinations of treatments in vitro, also establishing immortalized cell lines and culturing such cell lines, for example as host cells in genetic engineering and protein production, is much desirable.

### Disclosure of invention

It was therefore an object of the present invention to provide a culture medium improving the proliferation and survival of human epithelial cancer cells, in particular cells of head and neck cancer.

It was a further object to provide a method for the long time culturing and continuous proliferation of human squamous carcinoma cells.

These objects were met by the culture medium and the methods of the present invention as described below and in the claims.

The present invention concerns a culture medium for culturing human squamous carcinoma cells, that is characterized in that it comprises serum in trace amounts of not more than 0.015 % v/v, preferably about 0.01 % and at least one substance improving attachment to a surface.

In a preferred embodiment, the at least one substance allowing attachment is a combination of substances, in particular a combination of about 5 to 7 µg/ml fibronectin and about 10 to 15 µg/ml ascorbic acid whereby the culture surface is coated with collagen substratum. In a much preferred embodiment, the culture surface is collagen-type I coated and the culture medium comprises 5 µg/ml fibronectin and 10 µg/ml ascorbic acid.

The culture medium of the present invention usually comprises as basis a minimal medium suitable for culturing epithelial cells, such as MCDB-153 of Sigma, described in ^{21,22}. Such a suitable minimal medium - besides of general ingredients such as essential amino acids and trace elements, vitamins and other essential components - is characterized by a calcium concentration of about 0.07- 0.1 mM, in particular about 0.09 mM, a pH of about 7.2 - 7.4, and an osmolality of about 290 - 310 mOsm/kg. MCDB-153 medium is a modification of Ham's nutrient mixture F-12 with L-glutamine and 28 mM Hepes-buffer. It contains besides amino acids, trace elements, vitamins and other components. Usually the following amino acids in the mentioned concentrations are present in such a minimal medium:

| **Essential amino acids** ²¹ | **Concentrations g/l** ²¹ |
|---|---|
| L-Alanine | 0.00891 |
| L-Arginine•HCl | 0.2107 |
| L-Asparagine•H₂O | 0.01501 |
| L Aspartic Acid | 0.00399 |
| L-Cysteine•HCl•H₂O | 0.04204 |
| L-Glutamic Acid | 0.01471 |
| L-Glutamine | 0.8772 |
| Glycine | 0.00751 |
| L-Histidine•HCl•H₂O | 0.01677 |
| L-Isoleucine | 0.001968 |
| L-Leucine | 0.0656 |
| L-Lysine•HCl | 0.01827 |
| L-Methionine | 0.004476 |
| L-Phenylalanine | 0.004956 |
| L-Proline | 0.03453 |
| L-Serine | 0.06306 |
| L-Threonine | 0.01191 |
| L-Tryptophan | 0.00306 |
| L-Tyrosine•2Na•2H₂O | 0.00341 |
| L-Valine | 0.03513 |

whereby the amino acid concentration may vary in a range of ± 5 %.

Further ingredients that are present in such minimal media are:

| **Trace elements:** | **g/l** |
|---|---|
| Calcium chloride•2H₂O | 0.004411 |
| Potassium chloride | 0.11183 |
| Magnesium chloride•6H₂O | 0.122 |
| Sodium acetate | 0.30153 |
| Manganese sulfate | 0.000000151 |
| Molybdic acid [NH₄]₆ 4H₂O | 0.00000124 |
| Nickel chloride•6H₂O | 0.000000119 |
| Sodium selenite | 0.0000038 |
| Sodium metasilicate | 0.000142 |
| Stannous chloride•H₂O | 0.000000113 |
| Ammonium metavanadate | 0.000000586 |
| Sodium chloride | 7.599 |
| Sodium phosphate dibasic (anhydrous) | 0.284088 |
| CuSO₄ • 5H₂O | 0.00000275 |
| FeSO₄ • 7H₂O | 0.00139 |
| ZnSO₄ • 7H₂O | 0.000144 |

| **Vitamins:** | **g/l** |
|---|---|
| D-Biotin | 0.0000146 |
| D-Pantothenic acid hemicalcium | 0.000238 |
| Choline chloride | 0.01396 |
| Folic acid | 0.00079 |
| myo-Inositol | 0.01802 |
| Niacinamide | 0.00003663 |
| Pyridoxine • HCl | 0.00006171 |
| Riboflavin | 0.00003764 |
| Thiamine • HCl | 0.0003373 |
| Vitamin B₁₂ | 0.000407 |
| Adenine • HCl | 0.03088 |

| **Other components:** | **g/l** |
|---|---|
| DL-6,8-Thioctic acid | 0.0002063 |
| Putrescine • 2 HCl | 0.0001611 |
| Thymidine | 0.0007266 |
| D-Glucose | 1.081 |
| Hepes | 6.800 |
| Phenol Red • Na | 0.001242 |
| Pyruvic acid • Na | 0.0550 |

whereby the concentrations of trace elements, vitamins and other components can vary in a range of ± 5 %.

Powdered medium MCDB-153; complete with amino acids, trace elements, vitamins, and other components, is available by Sigma (Catalog "Compounds-cell culture reagents") No. M 7403/1999 for in vitro diagnostic use.

The culture medium preferably furthermore comprises ethanolamine, optionally and preferably together with phosphoethanolamine, as well as penicillin, streptomycin, fetal bovine serum, hydrocortisone, transferrin, 3,3',5-triiodo-L-thyronine (T3) or retinoic acid, bovine pituitary extract (BPE), insulin, and EGF (epidermal growth factor).

With regard to the known culture media, the following changes have been performed:

A basal medium was obtained by supplementing a minimal medium with ethanolamine, and optionally phosphoethanolamine. The phosphoethanolamine with a slower effect than ethanolamine improves the long-time effect, i.e. delays the time at which the culture medium has to be replaced. The inventive medium is obtained by adding to the basal medium a trace amount of serum, in particular fetal calf serum, and a substance promoting adhesion, in particular a combination of ascorbic acid and fibronectin. The trace amount of serum is at most 0.015 % v/v and preferably is about 0.01 %, and the combination of ascorbic acid and fibronectin as adhesion promoter is especially suitable for the culturing on collagen coated culture surfaces.

A suitable basis for the inventive culture medium, besides of the minimal medium MCDB-153, is a component deficient variation of Keratinocyte-SFM (GIBCO or Life Technologies) comprising the minimal medium MCDB-153, ethanolamine and phosphoethanolamine, but lacking hydrocortisone, EGF, T3 and insulin.

It has been found that the culturing can be improved by the addition of a trace amount of serum and by using an adhesion promoting system.

A further improvement can be obtained, if usually applied antimycotics are omitted in the culture medium whereby they are preferably applied at another stage within a process for preparing a cell culture, and if the concentration of penicillin and streptomycin are kept very low.

A further advantageous change is obtained by the specified transferrin concentration as well as the T3 concentration that is preferably enhanced from usually 20 nM to about 30 nM.

A preferred culture medium thus comprises the following ingredients in the following ranges or preferred concentrations, whereby the ingredients are divided into the ingredients comprised in the basal medium and the further ingredients by which the basal medium is supplemented to give preferred inventive "almost serum-free" media:

| Ingredient (basal medium) | Range | preferred concentration* |
|---|---|---|
| Calcium | 0.07-0.1 mM | 0.09 mM |
| Ethanolamine | 0.5 to 3 µg/ml | 2 µg/ml** |
| Phosphoethanolamine | 1 to 5 µg/ml | 2 Ag/ml** |
| essential amino acids, trace elements, etc. | see above | |
| Penicillin | 25-50 U/ml | 25 U/ml |
| Streptomycin | 25-50 µg/ml | 25 µg/ml |
| Hydrocortisone | 0.6-1.1 x 10⁻⁶M | 0.4 µg/ml 1.1 x 10⁻⁶ M |
| Transferrin (iron-transp.protein) | 5-10 µg/ml | 10 µg/mm |
| T3 or retinoic acid*** | 14-20 pg/ml 1-3 ng/ml | 20 pg/ml 3 ng/ml |
| BPE (bovine pituitary extract) | 20-30 µg/ml | 25 µg/ml |
| Ascorbic acid | 10-15 µg/ml | 10 µg/ml |
| Fibronectin | 5-7 µg/ml | 5 µg/ml |
| Insulin | 10-15 µg/ml | 10 µg/ml |
| EGF (epidermal growth factor) | 5-20 ng/ml | 20 ng/ml |
| Fetal bovine serum | at most 0.015 % | 0.01 % |

| | | |
|---|---|---|
| * Concentrations of the ingredients in the inventive, almost serum-free medium used in the examples. | | |
| ** If only ethanolamine is used, the amount can be left about the same, however the medium should more frequently be exchanged and preferably freshly prepared just prior to each exchange. | | |
| *** T3 can be replaced totally by retinoic acid, the range/concentration is valid for pure T3, or for pure retinoic acid, respectively. | | |

In view of found instabilities or reduced stabilities, it is preferred that the ingredients marked as further ingredients of the inventive culture medium are freshly added to the basal medium every 3-5 days (usually from stock solutions) and that the insulin, in particular bovine crystalline insulin, and the epidermal growth factor (EGF) are directly added to the cultures during medium change, said change being performed preferably every 3 days.

Thus, the present invention also comprises sets for the preparation of a culture medium according to the present invention. Such a set comprises as one component at least a basal medium, as a second component serum and as a third component attachment improving substance(s).Also further ingredients can be present as further components, and components can be present in pure form or as stock solutions.

The present invention furthermore comprises a method for culturing the above mentioned kinds of human carcinoma cells, that is characterized by culturing the cells in a culture medium according to the present invention. Preferably, the culture medium is changed every 3 days, whereby in particular the two hormones (insulin and EGF) found to have short half-life times in such culture media are replaced.

For subculturing the cells 80% confluency is necessary. The cell seeding density for successful cell proliferation, together with collagen-I coated culture dishes is about 10⁴/cm² area.

The method is especially suitable for epithelial cells originating from squamous cell carcinoma of the head/neck.

The present invention furthermore comprises a method for generating long-term epithelial cell cultures of human carcinoma cells. Said method is characterized in that it comprises the following steps in the following sequence:
a) fresh tissue is transferred into sterile transport-medium, preferably Dulbecco's modified eagle medium/nutrient mixture Ham's F-12 in a ratio of 3 : 1, complemented with antibiotics and antimycoticum, in particular 1 - 2 % v/v amphotericin-B,
b) the samples are washed once in concentrated antimycoticum-suspension, preferably Nystatin 10000 U/ml phosphate buffered sodium chloride (PBS) for about 2 to 3 minutes,
c) the samples are rinsed with sterile basal medium, cut into small explants and transplanted onto dishes providing a surface suitable for cell attachment, preferably collagen-I coated surfaces,
d) the attached explants are kept at roomtemperature for about 10 to 15 minutes and then are covered with a 1 : 1 mixture of embryonic fluid (Sigma) and fetal bovine serum, just one drop (about 20 µl), and incubated for about two hours at 37°C with a humidified atmosphere of 95 % air and 5 % CO₂.
e) to each of the attached explants is given culture medium according to the invention comprising all ingredients except insulin and EGF, preferably about 10 to 20 µl, and the cells are kept at 37°C and about 5% CO₂ for about 10 to 16 hours,
f) after said time, culture medium as specified under e) is added in regular intervals of preferably about 2 to 6 hours to a total volume of 500 µl after 48 hours (start of the time measurement after transplantation, i.e. step c)),
g) after about 72 hours (start of the time measurement after transplantation, i.e. step c)), the volume of the medium is extended to 2.5 ml by addition of medium specified under e) and addition of insulin and EGF.

At each medium change (usually every 3 days) used medium is replaced by fresh medium whereby the two hormones insulin and EGF are added to the cultures during the medium replacement.

The method of the present invention is especially suitable for establishing primary cell cultures but it can also be used to establish immortalized cell cultures suitable in e.g. genetic engineering.

Cell cultures obtained by the above described method are characterized by conservation of their original phenotype.

### Modes for carrying out the invention

Head and neck cancer includes tumors of the pharynx, larynx and mainly of the tongue in a progressive stage. The majority of these are squamous cell carcinoma, moderately differentiated with tumor grading II.

The tumor grade is a guide to patient prognosis. Despite advances in surgery, radiotherapy and chemotherapy, the 5-year survival rate has not changed significantly in 20 years ²³.

The clinical features of all patients are depicted in Table I. Tumor biopsies were obtained in each of them for in vitro processing.

The investigation of cell proliferation suggested that four factors are the most important ones in affecting the success of culture growth. These factors are shortly described below.

One of said factors is lack of tumor cell growth from the tissue placed in culture due to an inability of tumor cells to thrive because of inadequate nutrients or growth factors in the culture medium. Another factor is a sampling error in which no viable tumor was obtained, and/or a delay in processing the tissue that causes considerable cell death, even if a viable tumor was initially present.

Various media were tested for their ability to support successful outgrowth of human squamous epithelial cell carcinoma of head and neck using particle cultures. By said variation factors improving the survival and proliferation of cells in culture could be determined. Compositions of three different media (two comparative media and one inventive medium) are described more detailed in Table II.

The majority of tumor tissues (47%), processed in according to sections 1 and 2 of table II, showed either no cell outgrowth from the explants or epithelial growth was limited (13%), either by above mentioned failures, serum-containing media included, or by cell senescence at passages 2-3. It could be shown that serum is - as far as the presently concerned cells are at issue - erroneously viewed as a cell growth stimulant. It could be shown that survival and proliferation of squamous cell carcinoma rather is inhibited by serum. Said finding is in correlation with data for human bronchial epithelial cells for which it was shown that serum severely inhibits growth of some types of epithelial cells in defined media ²⁴. In the scope of the present invention it could, however, be shown that, although a usual amount of serum has an inhibiting effect, the presence of a minimal amount of serum, namely about 0.01 % v/v is very advantageous.

An additional cause of failure that was reported before is the overgrowth of promising epithelial cells by host fibroblasts ²⁵.

The fourth common cause of culture failure is contamination. Specimens can be lost both to preexisting contaminants in the tumor specimen and to infections, primarily fungal, that spread among the cultures because of inadequate isolation. Without specific provisions, several tumors (according to experiments about 27%) become contaminated with bacteria and/or yeast in vitro, a problem that was at least partially overcome in the scope of the present invention by brief pretreatment of the biopsies with an antimycoticum, such as nystatin, in a high concentration, in general in a concentration of about 10000 U/ml (see results in section 3 of Table II). Treatment of epithelial cells with nystatin over a longer period in order to avoid fungal infection causes cell senescence. However, for a short time, as it is disclosed for the present invention, it improves the overall rate of surviving cells.

Each of the above mentioned problems has been addressed in the scope of the present invention, at least with regard to preferred embodiments.

Under optimal culturing conditions (as used according to section 3 of Table II) cancer cells, in particular head /neck epithelial carcinoma cells that are preferably cultured according to the present invention, successfully grew from the explants (at least about 40%). Generally this growth became apparent after 5-7 days in culture.

As a further tool to optimize the inventive method, the cell seeding density is kept in a specific range. At a density of 10³ cells/cm², little growth occurred, and such a low cell density could be critical for cell survival.

Cell seeding densities of 5 to 10 x 10³ cells/cm² seem to be optimal and most consistently result in cell growth to confluence within 7-10 days after subculturing. It has, however to be understood that also cell seeding densities that are not optimal are possible and within the scope of the present invention.

The improvement obtained by the method of the present invention using the medium of the present invention can be shown by cytologic examination of growing primary cultures in almost serum-free, preferably hormone supplemented, medium of the present invention. Invert-phase microscopy showed compact cells with a higher nuclear: cytoplasma ratio compared to those with serum-supplemented medium as described in sections 1 and 2 in Table II. In serum-supplemented medium cells changed to large and multinucleated cells with subsequent loss of cell proliferation. In less than 10 days these cells sloughed off.

Deep frozen (-170°C) tissue-particles of biopsies from three patients could be explanted repeatedly as primary cultures in vitro with successful outgrowth of epithelial carcinoma cells if the method of the present invention was used.

Hitherto, 6 cell lines out of 15 patients were established using the optimized culture system of the present invention with trace amounts of serum, the "almost serum-free" culture medium. Besides of the above mentioned presence or absence, respectively, of components improving the cell proliferation, further factors have been examined. Growth activity and morphological features of differentiation are also dependent on ethanolamine, phosphoethanolamine, ion concentration, pH of culture media, physiological culture temperature and - as already mentioned - on high seeding densities.

As a further improvement substances improving the cell attachment are used. Collagen, a substantial component of the extracellular matrix ²⁶, is a preferred substance used to improve cell attachment and cell growth according to the present invention. A much preferred collagen is collagen-I. It is known that collagen promotes attachment of the cell membrane to the extracellular collagen matrix and attachment of cells through interaction with proteins, such as fibronectin, which are bound to the collagen matrix ²⁷. However, it could now be shown that such attachment can be improved by the simultaneous addition of ascorbic acid.

As a further additive, bovine pituitary extract has proven to be particularly valuable in the scope of the invention as an undefined supplement. When used in conjunction with other ingredients such as e.g. insulin, hydrocortisone and epidermal growth factor, it satisfies all of the additional growth requirements of epithelial cells, without promoting either terminal differentiation or fibroblastic overgrowth, both of which are - according to literature ^{28,29} - favored by serum supplementation. Nevertheless, the supplementation with bovine pituitary extract does not allow a total absence of serum. A trace amount of serum of at most 0.015 % v/v, preferably about 0.01% v/v is still necessary.

Further substances usable in the culture medium and thus the methods of the present invention are 3,3',5-triiodo-L-thyronine(T3) or retinoic acid. Retinoic acid and triiodo-L-thyronine(T3) have been found to be strong inhibitors of keratinization of squamous cell carcinoma. These factors almost completely inhibit stratification and keratinization of the cells at a concentration of about 10⁻⁹ M to 10⁻¹¹ M approximately the concentration required for both factors to maximally stimulate cell growth. Therefore it is likely that the growth-stimulating activities of retinoic acid and T3 are derived from their antikeratinization activities ¹⁴ . Wherever concentrations (10⁻¹¹ M) are mentioned in connection with T3, concentrations approximately to 10⁻⁹ M are valid for retinoic acid. Retinoic acid can be replaced with T3, but no further stimulation of growth is seen if T3 is added in addition to retinoic acid in the almost serum-free culture system.

For a culture system to have a maximum utility, the cultured cells should accurately reflect the initial properties of the tumor cells in vivo or at least - if used as host cells in genetic engeneering and/or protein production - all needed features. In the system here described, for all cell lines tested, phenotypic markers characteristic of epithelial cells and carcinoma, in particular squamous cell differentiation continued to be expressed in culture and, when examined microscopically, the morphology of cultured tumor cells was characteristic of squamous cell carcinoma.

Since for most applications it is of greatest importance that the cells in culture are, in fact, tumor cells, rigorous criteria were used to characterize the new tumor cell lines.

One important criterion is continuous growth. Each of the cell carcinoma lines examined has been passaged at least 12 times. Expression of appropriate differentiation features is another criterion ^{20,30}. Thus, histopathologic studies were performed to compare the morphologic characteristics of cells in culture with the surgical specimens. Remarkable concordance was found, even in identifying unique features for each cell line that were apparent in the tissue section.

The epithelial nature of cells, grown in hormone supplemented medium of the present invention, was verified by an indirect immunofluorescent staining of keratin filaments.

When epithelial cells of human squamous carcinoma derived from head and neck were cultured, the profile of keratin expression, seen in vivo, was maintained.

In order to get cell-outgrowth in a range of 5-10 %, it was found to be at least advantageous to explant minimum around hundred particles from each biopsy and much preferred to eliminate bacterial and fungal contaminations completely using at least one decontamination procedure, preferably at least two decontamination procedures, one for bacterial decontamination and one for fungal decontamination. By the performance of the decontamination in two steps the cell stress can be reduced and therefore the amount of surviving cells improved. In general the two steps are performed thus that in a first step fungal decontamination is obtained by washing the tissue with an antimycoticum in high concentration. The second decontamination step is preferably performed by adding antibiotics into the culture medium.

The invention is now further described by means of examples. These examples, however, are by no means intended to limit the scope of the invention.

### Examples

### General remarks

Malignant tissues were obtained from patients with advanced head and neck carcinoma after biopsy or resection of tumors. Squamous cell carcinoma was classified clinically according to the site of origin (S), tumor size (T), lymph node (N) and metastatic (M) involvement, and histopathological presentation (P).

The clinical features of all patients, tumors of which were studied in the scope of the following examples are depicted in Table I. Tumor biopsies were obtained in each of them for in vitro processing.

Fresh tissue was cut immediately in small samples by the pathologist and transferred in sterile transport-medium, complemented with antibiotics, for further processing. The transport-medium is not critical as long as the cells are sufficiently fast further processed. Suitable transport-medium is e.g. Dulbecco's MEM/nutrient Mix Ham's F-12 (3:1).

The culture media used in the examples are called basal medium and inventive and/or almost serum-free medium. They had the composition marked in the description as preferred composition.

### Example 1: Primary Cultures and Subcultures

### Cell preparation

Tumor specimens are processed as soon after removal as possible.

If necessary, a bulk of connective tissue was cut off from the epithelial tumor specimen and washed in concentrated nystatin-suspension (antimycoticum, 10000 U/ml phosphate buffered sodium chloride(PBS)) for 3 minutes, to avoid contaminations with fungi, which are known to be frequently present.

All specimens were rinsed several times with sterile basal medium, a MCDB-153 formula (Sigma) supplemented with ethanolamine and phosphoethanolamine, then minced into small pieces of approximately 1 mm² each with a sterile scalpel and transplanted onto collagen - type I coated 35 mm dishes or in Falcon inserts used with a companion TC-plate which were filled with 1 ml inventive culture medium.

Explants were transplanted and allowed to attach for 10 minutes at room temperature, then were covered with one drop (15-20 µl) of a 1:1 mixture of embryonic fluid (Sigma) and fetal bovine serum (Hyclone) and put for 2 hours at 37 °C in a humidified atmosphere of 95% air and 5% CO₂.

After 2 hours, 15-20 µl of a preferred inventive culture medium lacking insulin and EGF (details of composition above, further on referred to as almost serum-free or inventive culture medium) were added to each little piece and incubated overnight at 37 °C and 5 % CO₂. At least one hundred transplanted particles were necessary for successful outgrowth of sufficient cells for further cultures.

Until 48 hours after transplantation of the explants, almost serum-free culture medium lacking insulin and EGF was added to a total volume of 500 µl in steps of about 50 µl every 4 hours.

On the third day, after epithelial tumor particle transplantation, almost serum-free culture medium was added to the final volume of 2.5 ml, whereby for the first time insulin and EGF were added directly to the cultures. At each medium change (every 3 days) the medium was supplemented by adding directly to the tissue culture plate bovine crystalline insulin, 10 µg/ml (Calbiochem) and epidermal growth factor, 20 ng/ml (Life-Technologies).

After 5 days, clonal outgrowth of the epithelial tumor cells was recognizable. If 30 % cell confluency was reached, tissue particles were transplanted. For subculturing the remaining cells in the dishes, 80 % confluency was necessary. This offered a possibility of retransplantation of the particles (up to 7 times), to obtain continuously primary epithelial cells in passage zero.

### Culture medium

In the scope of the examples, as basis for the almost serum-free culture medium, original MCDB-153 (Sigma) with a calcium-concentration of 0.09 mM, a pH of 7.2-7.4 and an osmolality of 290-310 mOsm/kg, supplemented with ethanolamine and phosphoethanolamine, was used, or a component-deficient variation (lacking hydrocortisone, insulin and T3) of "Keratinocyte-SFM" (produced by GIBCO or Life Technologies).

The almost serum-free culture medium, comprised one of the above mentioned basal media and was supplemented with penicillin 25 U/ml and streptomycin 25 µg/ml (Hyclone) as well as 0.01% fetal bovine serum (Hyclone), hydrocortisone 0,4µg/ml (Calbiochem), human (iron-free) transferrin 10µg/ml (Sigma), 3,3',5-triiodo-L-thyronine 20pg/ml (Sigma), bovine pituitary extract 25µg/ml (Life Technologies), ascorbic acid 10µg/ml (Sigma) and fibronectin 5µg/ml (Boehringer-Mannheim).

The almost serum-free culture medium of the present invention of course can always be freshly prepared or mixed from stock solutions, whereby, also if stock solutions are used, insulin and epidermal growth factor have to be added directly to the culture dishes. In the scope of these examples stock solutions were used. A summary of the composition of concentrated stock solutions of medium supplements is presented in Table IV.

### Subcultivation of cultured human epithelial tumor cells

Using almost serum-free culture medium, cultured cells were rinsed with Versene (0.02% EDTA-solution), and then incubated at 37°C with 0.05% Trypsin/0.02% EDTA in PBS (Dulbecco's) for 5-10 minutes.

Cells were detached from the plastic surface by gentle pipetting. Trypsinization was terminated by addition of a twofold volume of Trypsin-inhibitor diluted in serum-free medium without supplements (soybean by Worthington: lmg/ml in 0.01 M phosphate-buffer, pH 6.5, with 0.15 M NaCl; inhibited 1.82 mg trypsin).

Harvested cells were dispersed by pipetting and counted with a hemocytometer. For cell-viability, Trypanblue-staining 0.4 % (0.1% stock-solution Sigma) was used. Cells were then plated on collagen-type I coated culture dishes in almost serum-free medium with an optimal cell-density of 10⁴/cm².

### Organic Substrates for Cell-Attachment

Due to the requirement of the transformed epithelial cells of head/neck tumors for collagen substratum collagen was used, mostly consisting of type I, originating from rat tail tendons, in form of a thin film of dried collagen in culture vessels (Becton Dickinson, coated Labware).

Fibronectin was added to the almost serum-free culture system, to improve cell-adhesion.

The working concentration of fibronectin in media was 5 µg/ml, used as a reconstituted sterile solution of lyophilized human plasma.

Fibronectin and ascorbic acid were added to stimulate the attachment to extracellular (basement membrane) collagen by the epithelial cells in monolayer cultures. A further component assumed to assist in the attachment is the epidermal growth factor.

### Example 2: Freezing/Rethawing of epithelial tumor tissue cells cultivated in almost serum-free media

"Cryo - SFM" (Promocell), a new formula for cryopreservation, as well as serum-free medium complemented with DMSO (5 to 10 % v/v) was used for cryo-preservation.

High initial vitality of the cells allowed a successful freezing and rethawing procedure. Only a small growth area was necessary to replate the cells.

Also freshly isolated and immediately deep frozen tissue samples from head/neck tumors were reexplanted in collagen-type I coated dishes after thawing with successful outgrowth of epithelial cells in the inventive almost serum-free medium described above.

Rethawed cells and explants were processed as described above. They showed at least as sucessful outgrowth as fresh tissue.

### Example 3: Immunocytochemical Staining

Keratin- and Vimentin filaments were examined in the six cell lines with extended cellular outgrowth of the tissue particles (shown in Table III).

### Technique:

Cells were grown on Biocoat-Culture Slides (Becton Dickinson-Collaborative Biomedical Products); a plastic chamber on a glass slide, collagen-type I coated, permitted culture in defined compartments. After culture was completed, the chamber was removed for cell-fixation.

### Cell-Fixation:

Cells were washed in phosphate-buffered saline (PBS) at room temperature, then fixed in 4 % formalin for 15-20 minutes following repeatedly washing with PBS and additional procedure with 100 % methanol for 10 minutes. This process ended with washing of the cells for 10 minutes again.

### Staining:

Slides were incubated with the following monoclonal mouse antibodies against intermediate filament proteins:

K 1, 5, 10, 14 (clone 34 β E 12, Enzo, Farmingdale, New York, USA), K 8 (clone 35 β H 11, Enzo), K 13 (clone Kₛ 13.1, Boehringer-Mannheim, Germany), K 19 (clone Kₛ 19.1, Progen, Heidelberg, Germany), K 20 (clone IT - Kₛ 20.8, Progen) and Vimentin (clone 3 B 4, Dako, Glostrup, Denmark).

For Ki-67 staining, monoclonal antibody MIB-1 (Dako) was used.

Primary antibodies were detected by a double sandwich technology using affinity purified goat antibodies against mouse immunoglobulins, followed by peroxidase labeled anti-goat immunoglobulin antibodies. The peroxidase mediated color reaction was performed with diaminobenzidine as substrate. Cells were counterstained with hemalum.

### Results:

All six cell lines expressed the keratin proteins K 1, 5, 10, 14 (typical for cornifying or squamous stratified epithelia) in all cells and K 13 (differentiation keratin typical for suprabasal cells of stratified squamous epithelia) to a variable proportion of cells (to the keratin proteins usually expressed by such cells, see ³¹ ). Simple epithelial keratins (K 8, 19) and vimentin were also expressed in most of the cells of every cell line. Keratin K 20 was consistently negative.

The extent of cells positive for the proliferation marker MIB-1 varied according to the growth ability of the individual cell lines.

Squamous cell carcinomas of the oral cavity, cultured according to the present invention exhibited an identical pattern of intermediate filament expression as described in ³². The presence of the differentiation markers K 1, 10 and especially of K 13 in these 6 cell lines confirmed their origin from squamous cell carcinomas ³³.

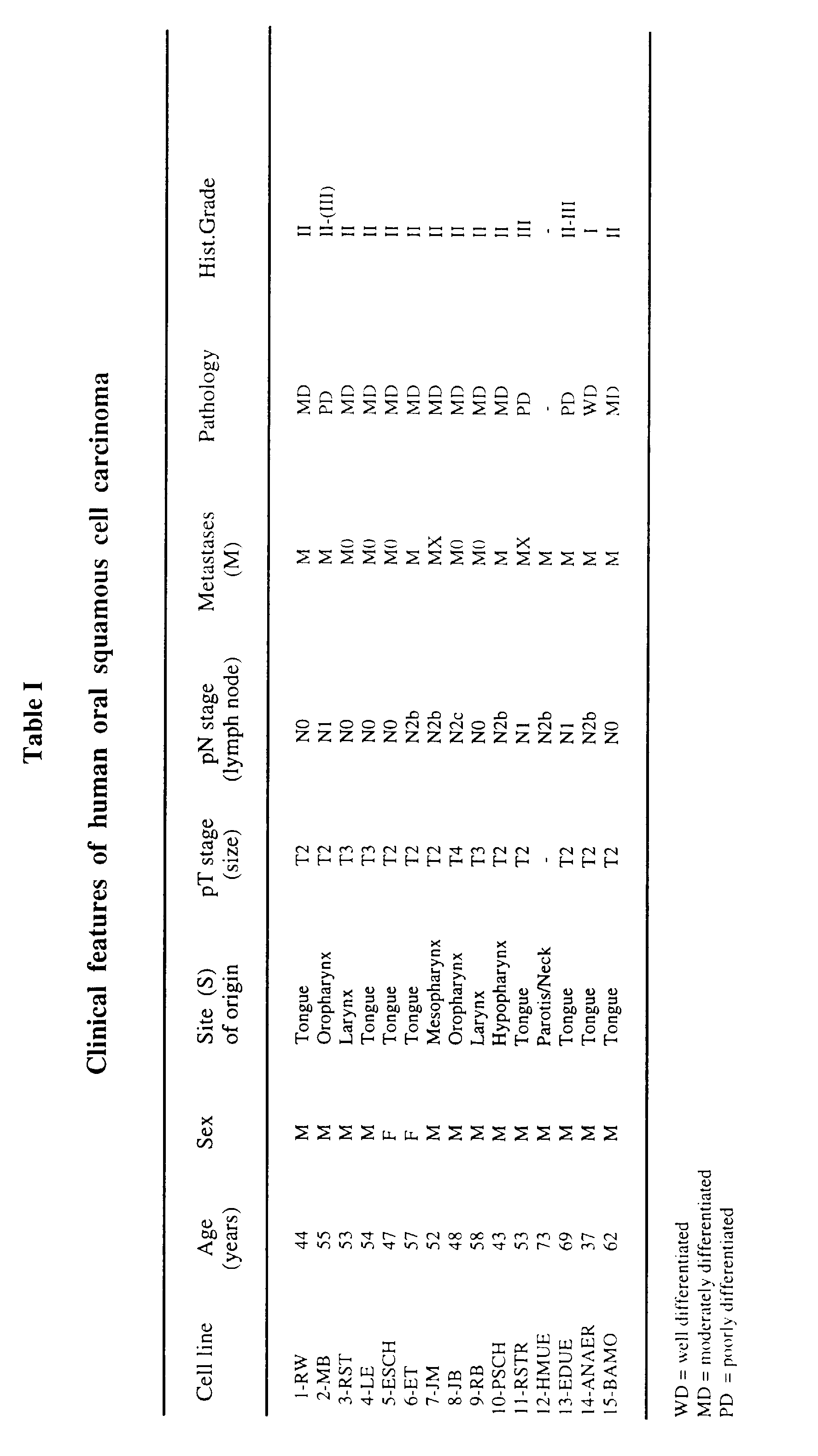

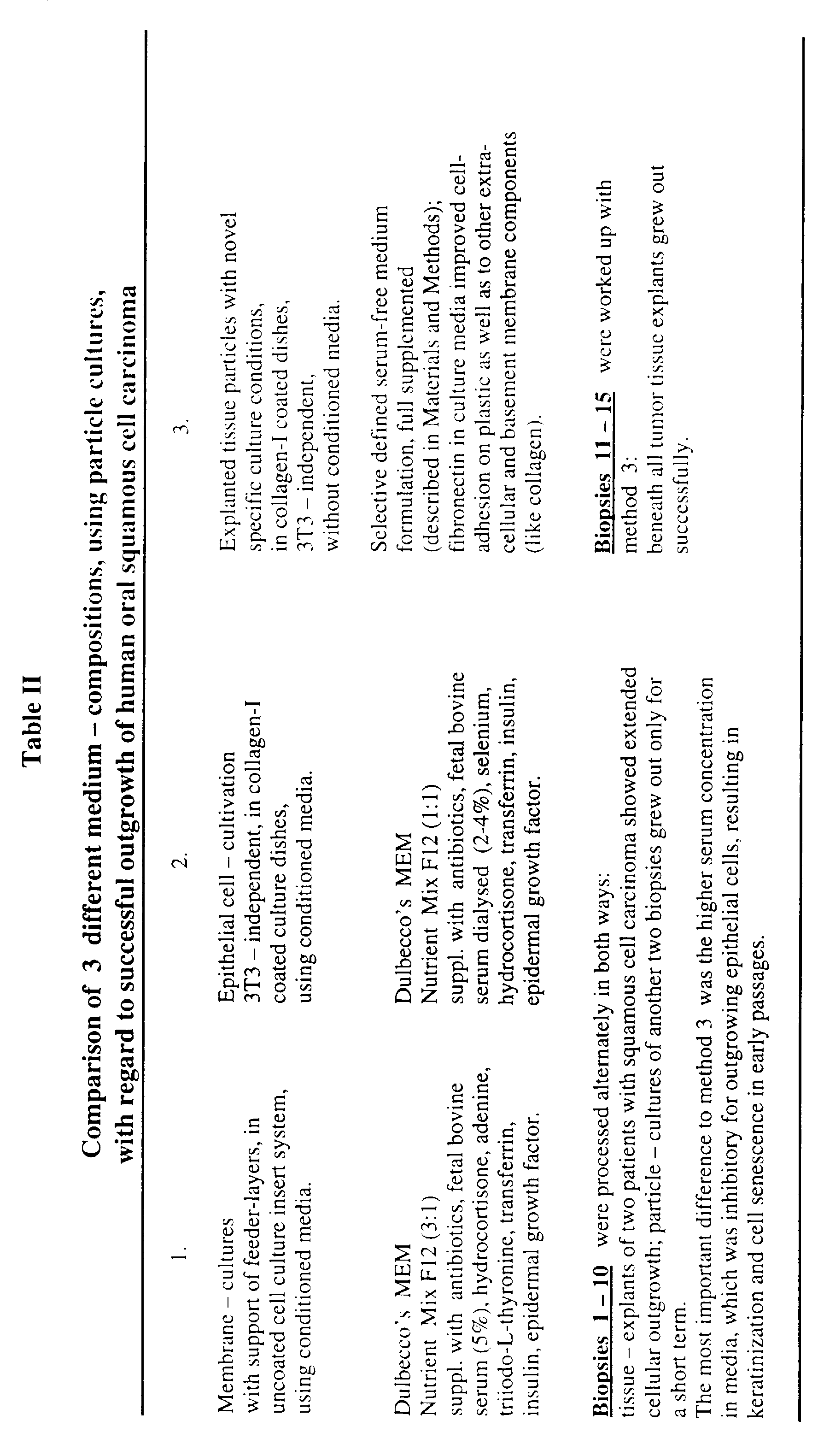

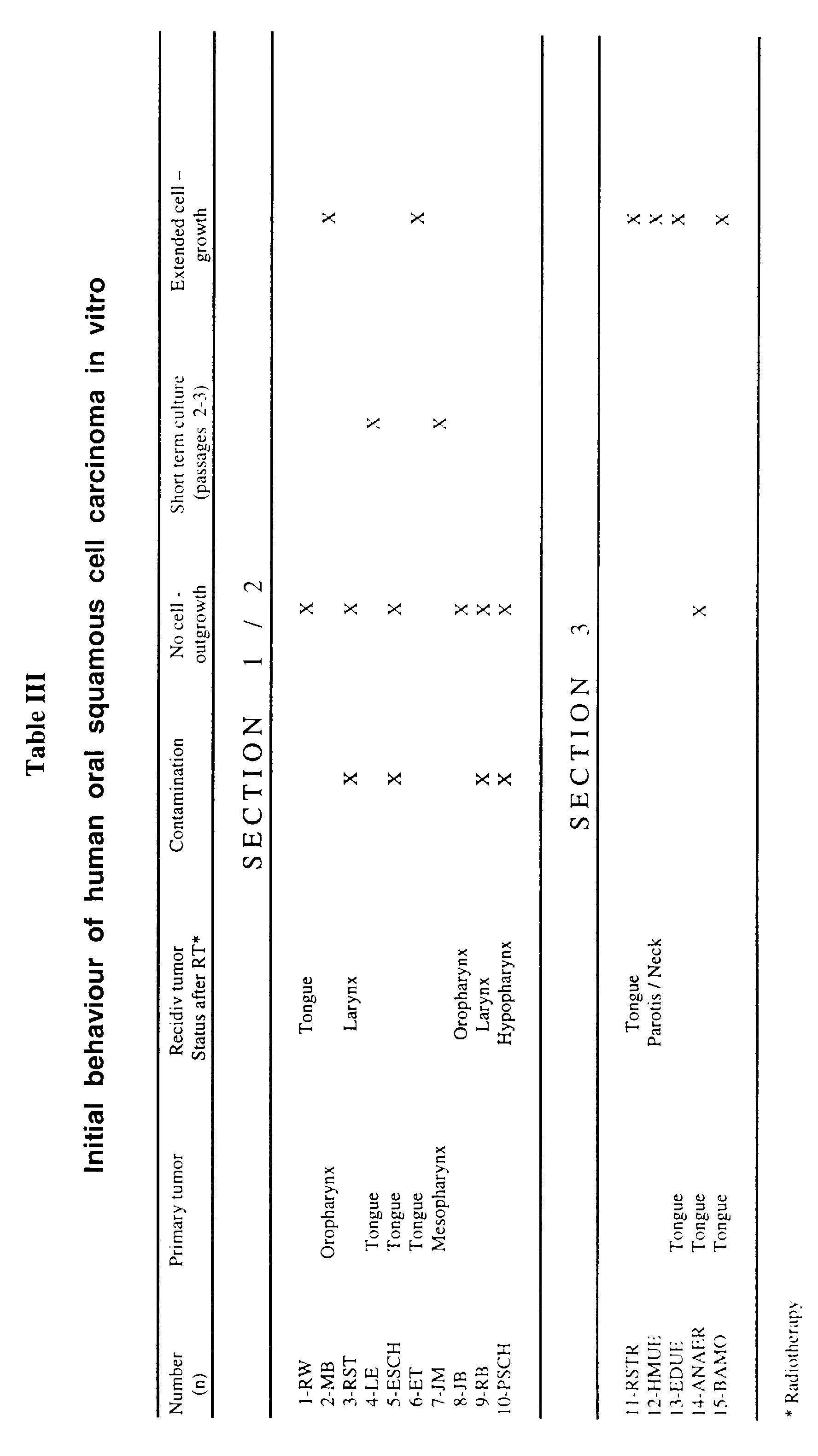

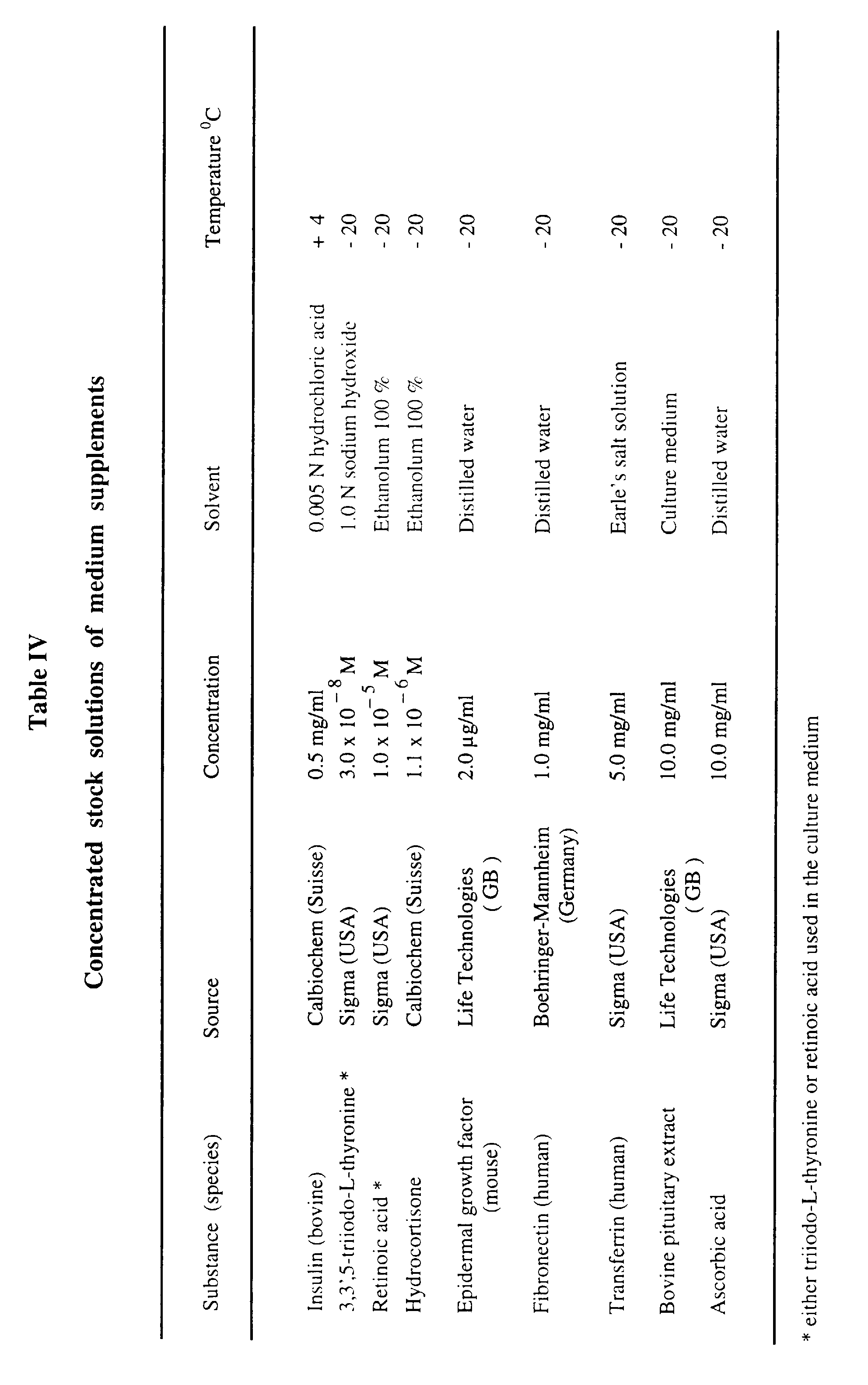

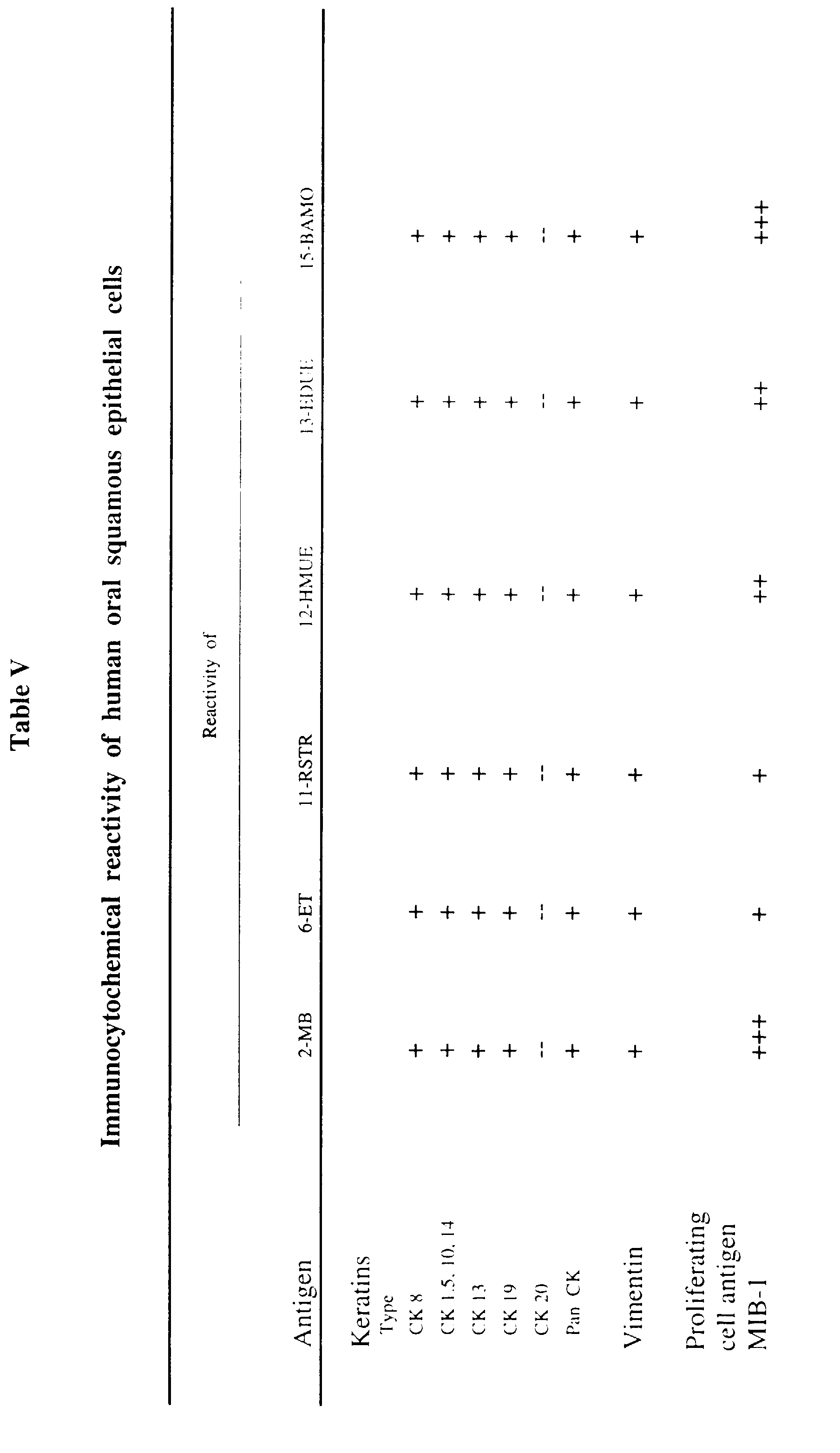

### References

1. Stell PM, McCormick MS. Cancer of the head and neck: are we doing any better?
   Lancet 1985;ii:1127.
2. Krause CJ, Carey TE, Ott RW, Hurbis C, McClatchey KD, Regezi JA. Human squamous cell carcinoma. Establishment and characterization of new permanent cell lines.
   Arch Otolaryngol 1981;107:703-10.
3. Gioanni J, Fischel J-L, Lambert J-C, et al. Two new human tumor cell lines derived from squamous cell carcinomas of the tongue:
   establishment, characterization and response to cytotoxic treatment. Eur J Cancer Clin Oncol 1988;24:1445-55.
4. Sacks PG, Parnes SM, Gallick GE, Mansouri Z, Lichtner R, Satya-Prakash KL,
   Pathak S, Parsons DF. Establishment and characterization of two new squamous cell carcinoma cell lines derived from tumors of the head and neck. Cancer Res 1988;48:2858-66.
5. Morris RJ, Tacker KC, Baldwin J-K, Fischer SM, Slaga TJ. A new medium for primary cultures of adult murine epidermal cells: application to experimental carcinogenesis. Cancer letters 1987; 34:297-304.
6. Burford-Mason AP, Irish J-C, MacKay AJ, Gullane PJ, Bassett R, Dardick I. Squamous cell carcinomas of the head and neck cultured in floating collagen gels: 1. The maintenance of stromal and epithelial elements in vitro without fibroblast overgrowth. Otolaryngol Head Neck Surgery 1997;116(2):213-22.
7. Taub M, Sato GH. Growth of functional primary cultures of kidney epithelial cells in defined medium. J Cell Physiol 1980;105:369-78.
8. Taub M. Importance of hormonally defined, serum-free medium for in vitro studies concerning epithelial transport. In: Tissue culture of epithelial cells. Plenum Press, New York 1985:255-77.
9. Handler J-S. Use of cultured epithelia to study transport and its regulation. J Exp Biol 1983; 106:55-69.
10. Gray TE, Thomassen DG, Mass MJ, et al. Quantitation of cell proliferation, colony formation, and carcinogen - induced cytotoxicity of rat tracheal epithelial cells grown in culture on 3T3 feeder layers. In Vitro 1983;19:559-70.
11. Boyce ST, Ham RG. Calcium - regulated differentiation of normal human epidermal keratinocytes in chemically defined clonal culture and serum-free serial culture. J Invest Dermatol 1983; 81:Suppl.33s-40s.
12. Ham RG. Selective media. In Cell Separation: Methods and Selected Applications, eds. Pretlow TG II and Pretlow T (Academic Press, New York) 1984;3:209-36.
13. Pardee AB, Cherington PV, Medrano EE. On deciding which factors regulate cell growth. In: Cell culture methods for molecular and cell biology. Eds. Barnes DW, Sirbasku DA, Sato GH. Alan R. Liss, Inc., New York 1984;11:157-66.
14. Barnes DW. Hormonally defined, serum-free media for epithelial cells in culture. In: Tissue culture of epithelial cells (edited by Taub M). Plenum Press, New York 1985:235-47.
15. Carney DN, Brower M, Bertness V, Oie HK. Selective growth of human small cell lung cancer cell lines and clinical specimens in serum-free medium. In: Cell culture methods for molecular and cell biology. Eds. Barnes DW, Sirbasku DA, Sato GH. Alan R. Liss, Inc., New York 1984;3:57-71.
16. Yamada KM. Cell surface interactions with extracellular materials. Ann Rev Biochem 1983;52:761-99.
17. Kleinman HK, Luckenbill - Edds L, Cannon FW, Sephel GC. Use of extracellular matrix components for cell culture. Analytical Biochemistry 1987;166:1-13.
18. Prime SS, Nixon SVR, Craine IJ, et al. The behaviour of human oral squamous cell carcinoma in cell culture. J Pathol 1990;160:259-69.
19. Miyazaki K, Masui H, Sato GH. Growth and differentiation of human bronchogenic epidermoid carcinoma cells in serum-free media. In: Cell culture methods for molecular and cell biology. Eds. Barnes DW, Sirbasku , GH. Alan R. Liss, Inc., New York 1984;7:83-94.
20. Fusenig NE, Breitkreutz D, Boukamp P. Differentiation potential of cancer cells. In: Human cancer in primary culture. A Handbook. Ed. Masters JRW. Kluwer Academic Publishers 1991;3:55-80.
21. Paul J. Cell and tissue cultures. Churchill Livingstone; Medical Division of Longman Group Limited; 5^{th} Edition; Edinburgh, London, New York 1975.
22. Ham RG. Importance of the basal nutrient medium in the design of hormonally defined media. In: Growth of cells in hormonally defined media. Book A. Eds. Sato GH, Pardee AB, Sirbasku DA. Cold Spring Harbor Conferences on Cell Proliferation 1982;9:39-60.
23. Henk J-M, Langdon J-D. Malignant tumours of the oral cavity. London: Edward Arnold 1985:71-9.
24. Lechner J-F, Haugen A, McClendon IA, Pettis EW. Clonal growth of normal adult human bronchial epithelial cells in a serum-free medium. In Vitro 1982;18:633-42.
25. Chen LL, Mann E, Greenberg B, et al. Removal of fibroblasts from primary cultures of squamous cell carcinoma of the head and neck. Journal of Tissue Culturing Methods 1993; 15:1-10.
26. Kleinman HK, Klebe RJ, Martin GR. Role of collagenous matrices in the adhesion and growth of cells. The Journal of Cell Biology 1981;88:473-85.
27. McKeown-Longo PJ. Fibronectin - cell surface interactions. Reviews of Infectious Diseases 1987;9(4):S322-34.
28. Hammond SL, Ham RG, Stampfer MR. Serum-free growth of human mammary epithelial cells: rapid clonal growth in defined medium and extended serial passage with pituitary extract. Proc Natl Acad Sci USA 1984;81:5435-9.
29. Gospodarowicz D. Purification of pituitary and brain fibroblast growth factors and their use in cell culture. In: Cell culture methods for molecular and cell biology. Eds. Barnes DW, Sirbasku DA, Sato GH. Alan R. Liss, Inc., New York 1984;12:167-97.
30. Kaartinen L, Nettesheim P, Adler KB, Randell SH. Rat tracheal epithelial cell differentiation in vitro. In Vitro Cell Dev Biol 1993;29A:481-92.
31. Moll R. Cytokeratins as markers of differentiation. Expression profiles in epithelia and epithelial tumors. Veroff Pathol 1993;142:1-119.
32. Störkel S, Moll R, Reichert T, Gillenkirch R, Spitz S, Wagner W. Expression von Intermediarfilament- und Onkogenproteinen in Plattenepithelkarzinomen der Mundhöhle. Korrelation zur Differenzierung. Dtsch Z Mund Kiefer GesichtsChir 1991;15:360-2.
33. Arany I, Adler-Storthz K, Chen Z, Tyring SK, Brysk H, Brysk MM. Differentiation markers in oral carcinoma cell lines and tumors. Anticancer Research 1997;17:4607-10.

## Claims

1. A culture medium for culturing human carcinoma cells, characterized in that it comprises a basal medium suitable for culturing epithelial cells with a calcium concentration of about 0.07-0.1 mM, a pH of about 7.2 - 7.4, and an osmolality of about 290 - 310 mOsm, kg, as well as ethanolamine, and in that the basal medium is supplemented with serum in an amount of at most about 0.015 % (v/v), preferably 0.01 %, and at least one substance improving attachment to a culture surface.

2. The culture medium of claim 1, wherein the at least one substance allowing attachment to a culture surface is a combination of substances, in particular fibronectin and ascorbic acid and the culture surface is collagen coated, in particular collagen-type I coated.

3. The culture medium of claim 2, characterized in that it comprises about 5 µg/ml fibronectin and about 10 µg/ml ascorbic acid.

4. The culture medium of anyone of claims 1 to 3, wherein the serum is fetal bovine/calf serum.

5. The culture medium of anyone of claims 1 to 4, characterized in that it comprises the following amino acids in the mentioned concentrations:
| **Essential amino acids** | **Concentrations g/l** |
|---|---|
| L-Alanine | 0.00891 |
| L-Arginine.HCl | 0.2107 |
| L-Asparagine•H₂O | 0.01501 |
| L-Aspartic Acid | 0.00399 |
| L-Cysteine•HCl•H₂O | 0.04204 |
| L-Glutamic Acid | 0.01471 |
| L-Glutamine | 0.8772 |
| Glycine | 0.00751 |
| L-Histidine•HCl•H₂O | 0.01677 |
| L-Isoleucine | 0.001968 |
| L-Leucine | 0.0656 |
| L-Lysine•HCL | 0.01827 |
| L-Methionine | 0.004476 |
| L-Phenylalanine | 0.004956 |
| L-Proline | 0.03453 |
| L-Serine | 0.06306 |
| L-Threonine | 0.01191 |
| L-Tryptophan | 0.00306 |
| L-Tyrosine•2Na•2H₂O | 0.00341 |
| L-Valine | 0.03513 |
and
| **Trace elements:** | **g/l** |
|---|---|
| Calcium chloride ·2H₂O | 0.004411 |
| Potassium chloride | 0.11183 |
| Magnesium chloride ·6H₂O | 0.122 |
| Sodium acetate | 0.30153 |
| Manganese sulfate | 0.000000151 |
| Molybdic acid [NH₄]₆ 4H₂O | 0.00000124 |
| Nickel chloride•6H₂O | 0.000000119 |
| Sodium selenite | 0.0000038 |
| Sodium metasilicate | 0.000142 |
| Stannous chloride•H₂O | 0.000000113 |
| Ammonium metavanadate | 0.000000586 |
| Sodium chloride | 7.599 |
| Sodium phosphate dibasic (anhydrous) | 0.284088 |
| CuSO₄ • 5H₂O | 0.00000275 |
| FeSO₄ • 7H₂O | 0.00139 |
| ZnSO₄ • 7H₂O | 0.000144 |
| **Vitamins:** | **g/l** |
|---|---|
| D-Biotin | 0.0000146 |
| D-Pantothenic acid hemicalcium | 0.000238 |
| Choline chloride | 0.01396 |
| Folic acid | 0.00079 |
| myo-Inositol | 0.01802 |
| Niacinamide | 0.00003663 |
| Pyridoxine • HCl | 0.00006171 |
| Riboflavin | 0.00003764 |
| Thiamine • HCl | 0.0003373 |
| Vitamin B₁₂ | 0.000407 |
| Adenine • HCl | 0.03088 |
| **Other components:** | **g/l** |
|---|---|
| DL-6,8-Thioctic acid | 0.0002063 |
| Putrescine • 2 HCl | 0.0001611 |
| Thymidine | 0.0007266 |
| D-Glucose | 1.081 |
| Hepes | 6.800 |
| Phenol Red • Na | 0.001242 |
| Pyruvic acid • Na | 0.0550 |
whereby the concentrations may vary in a range of ±5 %.

6. The culture medium of anyone of claims 1 to 5, characterized in that it furthermore comprises bovine pituitary extract, in particular in conjunction with insulin, hydrocortisone and epidermal growth factor.

7. The culture medium of anyone of claims 1 to 6, characterized in that it furthermore comprises penicillin, streptomycin, transferrin, and 3,3',5-triiodo-L-thyronine or retinoic acid.

8. The culture medium of anyone of claims 1 to 7, wherein the ingredients are used in the following concentrations:
| Ingredients | Range |
|---|---|
| Ethanolamine | 0.5-3 µg/ml |
| Phosphoethanolamine | 1-5 µg/ml |
| Penicillin | 25-50 U/ml |
| Streptomycin | 25-50 µg/ml |
| Hydrocortisone | 0.6-1.1 x 10⁻⁶ M |
| Transferrin (iron-transp.protein) | 5-10 µg/ml |
| T3 or retinoic acid | 14-20 pg/ml 1-3 ng/ml |
| BPE (bovine pituitary extract) | 20-30 µg/ml |
| Ascorbic acid | 10-15 µg/ml |
| Fibronectin | 5-7 µg/ml |
| Insulin | 10-15 µg/ml |
| EGF (epidermal growth factor) | 5-20 ng/ml |
| Fetal bovine serum | at most 0.015 % |
| Calcium | 0.07-0.1 mM |

9. The culture medium of claim 8, wherein the culture medium comprises:
| Ingredients | Concentration |
|---|---|
| Ethanolamine | 2 µg/ml |
| Phosphoethanolamine | 2 µg/ml |
| Penicillin | 25 U/ml medium |
| Streptomycin | 25 µg/ml medium |
| Hydrocortisone | 0.4 µg/ml medium 1.1 x 10⁻⁶ M |
| Transferrin (iron-transp.protein) | 10 µg/ml medium |
| T3 or | 20 pg/ml medium |
| retinoic acid | 3 ng/ml medium |
| BPE (bovine pituitary extract) | 25 µg/ml medium |
| Ascorbic acid | 10 µg/ml medium |
| Fibronectin | 5 µg/ml medium |
| Insulin | 10 µg/ml medium |
| EGF (epidermal growth factor) | 20 ng/ml medium |
| Fetal bovine serum | 0.01 % |
| Calcium | 0.09 mM |

10. A method for culturing human squamous carcinoma originating cells, characterized in that a culture medium according to anyone of claims 1 to 9 is used.

11. The method of claim 10, characterized in that the culture medium is changed every 3 days and that the cells are plated at a cell density of 10⁴/cm² area after subcultivation and that subcultivation is performed at about 80 % cell confluency.

12. The method of claim 10 or 11,
characterized in that the cells originate from squamous cell carcinoma of the head/neck area.

13. A method for generating long-term cell cultures of human carcinoma cells, characterized in that it comprises the following steps in the following sequence:
a) fresh tissue is transferred into sterile transport-medium complemented with antibiotics and antimycoticum,
b) the samples are washed once in concentrated antimycoticum-suspension for about 2 to 3 minutes,
c) the samples are rinsed with sterile basal medium, cut into small explants and transplanted onto dishes providing a surface suitable for cell attachment,
d) the attached explants are kept at room temperature for 10 to 15 minutes and then covered with a 1 : 1 mixture of embryonic fluid (Sigma) and fetal bovine serum, one drop of about 20 µl, and incubated for about two hours at 37°C with a humidified atmosphere of 95 % air and 5 % CO₂,
e) to each of the attached explants are given about 10-20 µl medium according to one of claims 1 to 9 but lacking insulin and EGF, and the cells were kept at 37°C and about 5% CO₂ for about 10 to 16 hours,
f) after said time, culture medium as specified under e) is added in regular intervals of preferably about 2 to 6 hours to a total volume of 500 µl after 48 hours,
g) after about 72 hours, the volume of the medium is extended to 2.5 ml by addition of medium specified under e) and addition of insulin and EGF.

14. The method of claim 13 wherein the steps are performed with the following characteristics:
a) the sterile transport medium is Dulbecco's modified eagle medium/nutrient mixture Ham's F-12 (3:1) complemented with 1 - 2 % v/v amphotericin-B as antimycoticum,
b) the concentrated antimycoticum-suspension is Nystatin 10000 U/ml phosphate buffered sodium chloride (PBS),
c) the surface suitable for cell attachment, is a collagen-I coated surface,
e) the medium lacking insulin and EGF otherwise corresponds to the one of claim 9,
f) the culture medium is the medium of claim 9 but lacking insulin and EGF.

15. Use of a culture medium of anyone of claims 1 to 9 for culturing human epithelial cells, in particular human squamous carcinoma cells derived from head/neck cancer.

16. A set for the preparation of a culture medium according to anyone of claims 1 to 9 comprising as one component at least a basal medium, as a second component serum and as a third component attachment improving substance(s).

17. The set of claim 16, characterized in that it comprises further components for further ingredients.

18. A cell culture characterized in that it comprises epithelial cancer cells that are conserving their initial phenotype and that are obtainable according to the method of one of claims 10 to 14.
